# EUROPÄISCHE PATENTSCHRIFT

(11) **EP 1 539 006 B1**
(45) Veröffentlichungstag und Bekanntmachung des Hinweises auf die Patenterteilung: **17.12.2008**
(21) Anmeldenummer: 03794925.2
(22) Anmeldetag: 22.08.2003
(51) Int. Cl.: A61B 17/72, A61B 17/68

(54) **INTRAMEDULLÄRER OSTEOSYNTHESE-NAGEL ZUM VERSORGEN VON RÖHRENKNOCHENFRAKTUREN**
INTRAMEDULLARY OSTEOSYNTHESIS PIN FOR THERAPY OF LONG BONE FRACTURES
CLOU D'OSTEOSYNTHESE CENTRO-MEDULLAIRE SERVANT A SOIGNER DES FRACTURES DES OS LONGS

(30) Priorität: 24.08.2002 DE 20213235 U
(43) Veröffentlichungstag der Anmeldung: 15.06.2005
(73) Patentinhaber: Tantum AG, 24537 Neumünster (DE)
(72) Erfinder: ROTH, Wolfgang, 66802 Überherrn (DE); HARDER, Hans, E., 24253 Probsteierhagen (DE)
(74) Vertreter: Wenzel & Kalkoff
(86) Internationale Anmeldenummer: PCT/EP2003/009353
(87) Internationale Veröffentlichungsnummer: WO 2004/024012

(56) Entgegenhaltungen:
- FR-A- 2 783 699
- GB-A- 1 428 653
- US-A- 5 100 405

## Beschreibung

Die Erfindung betrifft einen intramedullären Osteosynthese-Nagel zum Versorgen von Röhrenknochenfrakturen, umfassend einen bohrungsfrei in Knochenmark einbringbaren, sich in Axialrichtung lang erstreckenden Schaft mit einem Fußteil und einem Kopfteil, ein zum Verriegeln des Nagels im Knochen mit selbstschneidendem Außengewinde versehenes Kappenelement mit einer Aufnahme, mittels der es frei drehbar um die Längsachse des Schaft-Kopfteils auf diesen aufgesetzt ist, und eine Sicherungs-Verbindung, die das Kappenelement frei von Schraubverbindung unverlierbar mit dem Nagelschaft derart verbindet, daß das Kappenelement gegen Abziehen von dem Nagelschaft gesichert ist, wobei zwischen dem Kappenelement und dem Kopfteil des Schafts eine die freie relative Drehung zwischen den beiden Teilen zulassende Steckverbindung mit einem Steckabschnitt des Schaft-Kopfteils und zugeordneter Steckaufnahmen des Kappenelements ausgebildet ist. Es handelt sich um einen Marknagel zum Stabilisieren und/ oder Ausrichten von Röhrenknochen-Fragmenten nach oder während Reposition. Insbesondere wird der Knochennagel zur Frakturheilung von Fibula, Tibia, Ulna oder Radius verwendet. Je nach Anwendung weist der Nagel einen Durchmesser in der Größenordnung von 2 bis 6 mm auf, und er ist ca. 15 bis 40 cm lang. Der Nagel wird ohne besondere Bohrung im Knochen in das Knochenmark eingebracht und durch Endstellen der Knochenfrakturelemente hindurchgesetzt. Das Kappenelement setzt den Nagel in axialer Richtung fest und sichert ihn gegen Migration. Der Nagel unterstützt den normalen biologischen Heilungsprozeß des Knochens.

Aus FR 2 749 157 ist ein gattungsgemäßer Osteosynthese-Nagel bekannt. Eine mit selbstschneidendem Außengewinde versehene Kappe ist auf einen im Durchmesser zurückspringenden Steckabschnitt eines Nagels aufgesetzt. Der Nagel wird in unverlierbarer Verbindung mit der Kappe in den Knochen eingebracht. Die gegen Axialspiel gesicherte Nagelkappe ist um den zylindrischen Steckabschnitt frei drehbar gelagert. Der Nagelkopf weist einen verformten, gegenüber dem Steckabschnitt verdickten radialen Vorsprung auf, der in eine korrespondierende Senkung am Kappenende einfaßt. Die Anbringung von Drehwerkzeug, das den Kappenkopf überfassen muß, über Kerben in dem Außenrand der Senkung ist erschwert. Insbesondere beim Einbringen des Nagels kann der Kopfvorsprung des Nagels verformt und beschädigt werden, wodurch die Drehlagerung der Kappe zerstört wird.

Aus EP 0 547 101 ist ein Nagel mit einer ein Schneid-Außengewinde aufweisenden Kappe bekannt, die zur Verankerung des Nagels erst angebracht wird, wenn dieser bereits gesetzt worden ist. Die Kappe wird mittels Schraubverbindung mit dem Nagel verbunden, und zwar über ein feines Außengewinde oder Innengewinde am Nagelende. Abgesehen davon, daß die Schraubverbindung, die die Verankerungskappe in ihrer Befestigungsposition an dem Nagel sichert, freie Drehung der Kappe um die Nagelachse nicht zuläßt, muß das Nagelende als besonders zu fertigender Teil einer Gewindeverbindung ausgeführt werden. Das Innengewinde am Nagel erfordert die zusätzliche Verwendung einer Schraube. Die Gewinde müssen präzise gefertigt sein. Damit sind Herstellungsaufwand und entsprechende Herstellungskosten verbunden.

Ziele der Erfindung bestehen darin, einen intramedullären Osteosynthese-Nagel zum Versorgen von Röhenknochenfrakturen zu schaffen, der einfach bauen, einfach in der operativen Anwendung und kostengünstig in der Herstellung sein soll. Der Nagel soll in axialer Richtung wirksam verriegelt und gegen Migration gesichert werden können.

Die Ziele der Erfindung werden in Verbindung mit den Merkmalen des eingangs genannten Osteosynthese-Nagels dadurch erreicht, daß das Kappenelement einen Kopfteil aufweist, der frei von der Sicherungs-Verbindung bleibt, wobei die Sicherungs-Verbindung an einer von dem Kopfende des Nagelschafts in axialer Richtung zum Schaft-Fußende hin beabstandeten Umfangsstelle des Schaft-Steckabschnitts zwischen diesem und dem Kappenelement angeordnet und ausgebildet ist. Mit der Steckverbindung einerseits und der Sicherungs-Verbindung andererseits sind Kappenelement und Nagel ohne Schraubverbindung sowie frei von besonderen Verbindungselementen am Kopfende des Nagels miteinander unverlierbar verbunden. Kappenelement und Nagel sind einfach gestaltet. Die koaxiale Drehverbindung von Schaft und Kappenelement ist im Bereich der Kopfenden der beiden Teile vor Beschädigung geschützt, und zwar insbesondere wenn der Schaft in Verbindung mit dem Kappenelement eingebracht oder entfernt wird. Der Kopf des Kappenelements läßt sich einfach und robust zum Anschluß mit Drehwerkzeug ausbilden. Das Nagelschaftende bleibt frei von Formungen, Vorsprüngen od.dgl.. Dadurch werden Herstellung und Anwendung des Knochennagels besonders vereinfacht. Die Verbindung zwischen dem Kappenelement und dem Nagelschaft ist ausschließlich durch die Steckverbindung in Kombination mit der Sicherungs-Verbindung hergestellt. Kappenelement und Nagelschaft, die keine aufwendige Anpassung aneinander erfordern, bilden einen als unverlierbare Einheit zur Verfügung stehenden Nagel mit vorzugsweise unlösbar aneinandergesetzten Teilen. Die Einheit ist einfach bereitzuhalten und zu handhaben. Operative Maßnahmen sind vereinfacht. Der Nagel kann nicht nur einfach gesetzt, sondern in dauerhafter Verbindung des Kappenelements mit dem Nagelschaft auch besonders einfach entfernt werden. In Steckpassung wird das kopfseitige Nagelende genau in Position gebracht und gehalten, so daß der Nagel über das Außengewinde des Kappenelements im Knochen verriegelt wird. Die beiden Teile sind über den gesamten Schraubweg des Kappenelements relativ leichtgängig koaxial drehbar. Das erfindungsgemäß vorgesehene Kappenelement gewährleistet eine Verriegelung, die Reposition unterstützt, die Lage aneinandergefügter Knochenfragmente stabilisiert und Migration vermeidet.

Zweckmäßig kann das Kappenelement einen außerhalb des Kopfteils des Nagelschafts sich erstreckenden schaftfreien Kappen-Kopfteil zur Verbindung mit Drehwekzeug sowie die den Schaft-Steckabschnitt aufnehmende Steckaufnahme aufweisen. Das am Kopfteil des Nagelschaftes überstehende Kappen-Kopfteil kann vorteilhaft als Kappen-Hohlkopf mit mehrkantiger Aussparung zur Verbindung mit Drehwerkzeug ausgebildet sein. Von Bedeutung ist dabei, daß sowohl der Kappen-Hohlkopf als auch die Steckaufnahme frei von Gewindeelementen bleiben.

Die Sicherungs-Verbindung kann wenigstens einen an dem Kappenelement angeordneten Vorsprung und eine an dem Nagelschaft ausgebildete zugeordnete Ausnehmung aufweisen.

Gemäß besonderer Ausgestaltung der Erfindung kann die von dem Schaft-Kopfende beabstandet angeordnete Sicherungs-Verbindung dadurch gebildet sein, daß sie wenigstens ein einen Verbindungs-Eingriff zwischen dem Kappenelement und dem Nagelschaft herstellendes, von dem Schaftkopfende beabstandetes Federelement umfaßt, dessen Dimension in der Schaft-Axialrichtung kurz gegenüber der Stecklänge des Nagelschafts in der Steckverbindung ist. Ein solches Federelement kann zweckmäßig ein Bördelrand sein, der an dem dem Schaft-Fußteil zugewandten Ende des Kappenelements ausgebildet ist und in eine zugeordnete, vom Schaft-Kopfende entfernt gelegene sowie am Nagelschaft ausgebildete Ausnehmung eingreift, um vorzugsweise eine unlösbare Verbindung herzustellen. Form und Herstellung des erfindungsgemäßen Nagels dieser Gestaltung sind besonders einfach. Insbesondere in Verbindung mit dem Bördelrand besteht eine Ausgestaltung darin, daß sich der Nagelschaft längs einer Nut konisch derart erweitert, daß ein Axialbewegung des Kappenelements in Richtung zum Schaft-Fußende begrenzender Anschlag ausgebildet ist. Allgemein kann die Sicherungs-Verbindung des erfindungsgemäßen Nagels mit Spielpassung derart ausgebildet sein, daß in unverlierbarer Steckverbindung in Axialrichtung des Nagelschafts relatives Bewegungsspiel zwischen dem Kappenelement und dem Nagelschaft bleibt. Zweckmäßig kann am Nagelschaft eine Nut ausgebildet sein, die in Schaft-Axialrichtung eine Axialspiel zulassende Breite aufweist. Durch Axialspiel, das nur ganz geringfügig sein kann, kann die Frakturheilung gegebenenfalls gefördert werden. Insbesondere wird bei Bedarf geringfügige Verschiebung des Nagelschafts in Richtung auf sein Fußende zugelassen. Um Axialspiel zu vermeiden, kann die Einstecklänge der Steckverbindung zwischen einem Federelement, zweckmäßig einem Bördelrand, und dem Boden der Steckaufnahme so bemessen sein, daß der Steckabschnitt des Nagelschafts in Axialrichtung relativ unverschiebbar zu dem Kappenelement in dieses eingreift.

Gemäß einer Ausgestaltung der Erfindung ist die Sicherungs-Verbindung durch wenigstens ein sowohl in den Nagelschaft als auch in das Kappenelement eingreifendes Federelement gebildet. Besonders vorteilhaft kann die Sicherungs-Verbindung im Bereich des äußeren Drittels der Steckaufnahme der Steckverbindung ausgebildet sein.

In bevorzugter erfindungsgemäßer Ausgestaltung wird das Kappen-Kopfteil als Hohlkopf mit Durchgangsloch zwischen dem Kappen-Kopfteil und der Steckaufnahme ausgebildet. Zweckmäßig kann das Durchgangsloch einen der Steckaufnahme zugewandten umlaufenden Rand aufweisen, der in der Steckverbindung einen Bodenanschlag für die Kopf-Endfläche des Nagelschafts bildet. Mit solchem schmalem Rand ist die Drehverbindung von Kappenelement und Nagelschaft selbst dann noch leichtgängig, wenn das Kappenelement mit erhöhter Druckkraft gegen die Endfläche des Nagelschafts arbeitet.

Ganz allgemein können das Kappenelement in erfindungsgemäßer Ausgestaltung einen den Kappen-Kopfteil von der Steckaufnahme abgrenzenden Boden aufweisen und eine Nut/Feder-Verbindung der Sicherungs-Verbindung so ausgebildet sein, daß die Endfläche des Schaftkopfes an dem Boden zur Stoßanlage kommt. Dadurch kann der Nagelschaft durch Druckkraft auf das Kappenelement zum Verriegeln in den zu versorgenden Knochen eingebracht werden.

Nach der Erfindung kann der Schaft-Kopfteil wenigstens zwei Steckabschnitte und das Kappenelement für jeden Steckabschnitt eine zugeordnete Steckaufnahme aufweisen, wobei die Sicherungs-Verbindung vorteilhaft eine in der Steckverbindung in Schaft-Axialrichtung Spiel belassende Nut aufweist.

Besonders vorteilhaft ist es auch, daß das Kappenelement längs seiner gesamten Axiallänge mit Außengewinde versehen sein kann. Man erreicht, zweckmäßig in Verbindung mit relativ grobem, innige Verbindung herstellendem Außengewinde, besonders wirksame Verriegelung. Von besonderem Vorteil ist es, daß der Kappen-Hohlkopf eine umlaufende Kappenwand aufweisen kann, die stärker als die umlaufende Wand der angrenzenden Steckaufnahme ausgebildet ist. Dadurch kann die Verbindung mit in den Kappen-Hohlkopf eingreifendem Drehwerkzeug besonders robust ausgeführt werden.

Nach einer weiteren vorteilhaften Ausgestaltung der Erfindung kann der Nagelschaft wenigstens in dem größten Teil der Steckverbindung den gleichen Querschnitt wie in einem an die Steckverbindung anschließenden, zum Schaft-Fußende hin gelegenen langgestreckten Schaft-Abschnitt aufweisen. Dadurch sind Bauform und Herstellung des Nagels besonders einfach.

Unteransprüche sind auf die genannten und noch andere zweckmäßige und vorteilhafte Ausgestaltungen der Erfindung gerichtet. Besonders zweckmäßige und vorteilhafte Ausbildungsformen oder -möglichkeiten der Erfindung werden anhand der folgenden Beschreibung der in der schematischen Zeichnung dargestellten Ausführungsbeispiele beschrieben. Es zeigen
- Fig. 1: in Längsansicht einen erfindungsgemäßen Osteosynthese-Nagel und
- Fig. 2 und 3: jeweils in Teil-Längsansicht in Ausführungsbeispielen die Kappenelement-Verbindung erfindungsgemäßer Marknägel.

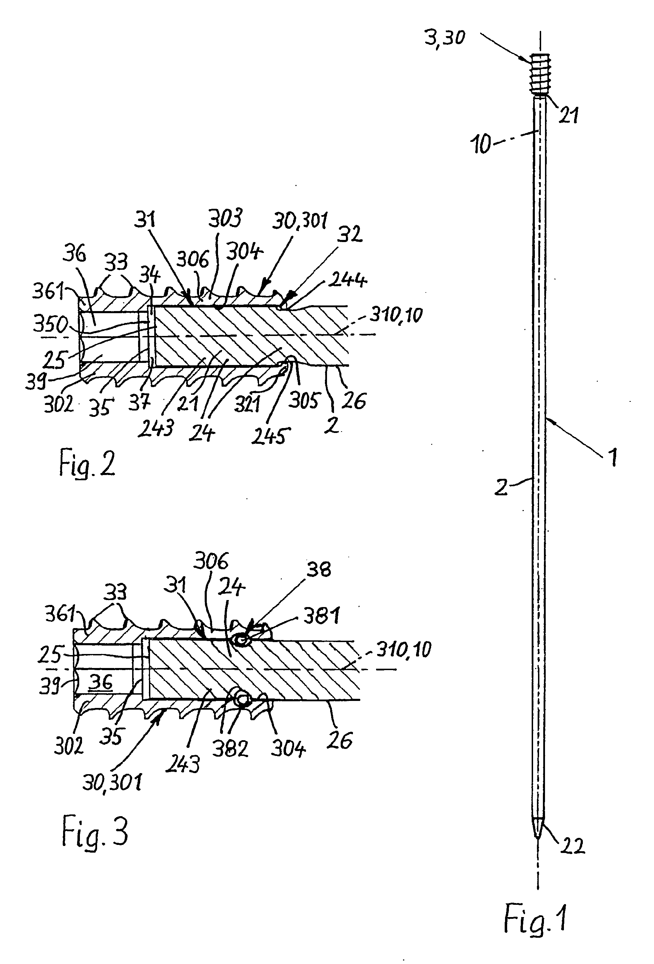

Der in Fig. 1 dargestellte Osteosynthese-Nagel 1, der sich in Richtung seiner Längsachse 10 lang erstreckt, umfaßt einen Schaft 2 und ein Verriegelungsmittel 3, das durch ein auf den Kopfteil 21 des Schafts aufgesetztes Kappenelement 30 gebildet ist. Das Fußende 22 des Schafts 2 ist mit einer geraden Spitze versehen, um den Nagel 1 einfach einbringen und durch Endstellen aneinanderzufügender Knochenfragmente eines Röhrenknochens setzen zu können. Erfindungsgemäße Nägel können auch gebogene Spitzen aufweisen. Das Kappenelement 30 und der Nagelschaft 2 sind zum Beispiel aus Titanlegierungen hergestellt. Der Schaft 2 des Nagels 1 mit gerader Spitze kann an seinem Fuß mit geeignetem Werkzeug auf wählbare Gesamtlänge abgelängt werden.

In den Ausführungsbeispielen der Fig. 1 bis 3 ist das Kappenelement 30 durch eine Aufsatzschraube 301 gebildet. Diese wird als kreiszylindrisches Drehteil mit Längsachse 310 hergestellt. Über ihre gesamte Axiallänge ist sie mit einem relativ groben, selbstschneidenden Außengewinde 33 versehen. Sie weist einen Hohl-Kopfteil 302 und einen sich ca. zweimal länger erstreckenden Steckteil 303 auf. In diesen ist eine sich langerstreckende kreiszylindrische Steckbohrung 34 eingebracht, die eine Steckaufnahme 304 bildet. Diese geht in ein Durchgangsloch 36 des Kopfteils 302 über. Der Radialdurchmesser der Steckaufnahme 304 ist etwas größer als der Durchmesser des Loches 36, so daß der Boden 35 der Steckaufnahme 304 durch einen umlaufenden Rand gebildet ist. Man erkennt, daß aufgrund des umlaufenden Bodenrandes der Kappen-Hohlkopf eine umlaufende Kappenwand 361 aufweist, die stärker als die umlaufende Wand 306 der angrenzenden Steckaufnahme 304 ist. Das Durchgangsloch 36 des Kopfteils 302, das sich schaftfrei außerhalb des Kopfendes 25 des Nagelschafts 2 erstreckt, weist eine zum Beispiel sechskantige Aussparung 39 zum Anschluß von Drehwerkzeug auf.

An seinem kopfseitigen Ende weist der Nagelschaft 2 einen Steckabschnitt 24 auf, der mit dem Steckteil 303 korrespondiert. Zur Ausbildung einer Steckverbindung 31 des erfindungsgemäßen Nagels 1 ist der Nagelschaft 2 mit seinem Steckabschnitt 24 in das Steckteil 303 hineingesteckt. Zwar ist zwischen dem Steckteil 303 und dem Steckabschnitt 24 eine Steckpassung hergestellt, doch sind an diese keine besonders hohen Genauigkeitsanforderungen zu stellen.

Wie aus den Ausführungsbeispielen der Fig. 2 und 3 ersichtlich, ist das Kappenelement 30 erfindungsgemäß unverlierbar und unlösbar mit dem Kopfteil 21 des Nagelschafts 2 verbunden. In Kombination mit der Steckverbindung 31 ist eine besondere Sicherungs-Verbindung 32 bzw. 38 ausgebildet. Diese ist jeweils derart, daß das auf den Schaft 2 aufgesteckte Kappenelement 30 in der Steckverbindung 31 um die zusammenfallenden Achsen 10, 310 drehbar bleibt. Jede Sicherungs-Verbindung 32, 38 verbindet das Kappenelement 30 frei von Schraubverbindung unverlierbar dauerhaft mit dem Nagelschaft 2. Einerseits ist das Kappenelement 30 gegen Abziehen von dem Nagelschaft 2 gesichert. Andererseits ist die Steckverbindung 31 derart, daß die Axialposition des Kappenelements 30 in Richtung auf das Fußende 22 des Nagelschafts 2 mit Anschlägen 350 bzw. 382 bestimmt ist.

In jedem Fall bleibt erfindungsgemäß der Kappen-Kopfteil 302 frei von der Sicherungs-Verbindung 32 bzw. 38. Wie aus Fig. 2 und 3 ersichtlich, ist die Sicherungs-Verbindung 32 bzw. 38 an einer von dem Kopfende 25 des Nagelschafts 2 in axialer Richtung zum Schaft-Fußende 22 hin entfernt gelegenen Umfangsstelle des Schaft-Steckabschnitts 243 zwischen diesem und dem Kappenelement 30 angeordnet und ausgebildet.

In Fig. 2 umfaßt die Sicherungs-Verbindung 32 einen am freien Rand der Steckaufnahme 304 zur Achse 310 abgebogenen umlaufenden Bördelrand 321 und eine zugeordnete am Nagelschaft 2 umlaufende Nut 245, in die der Bördelrand 321 als Vorsprung oder erhabenes Element eingreift. Der Bördelrand 321 ist an dem freien Rand der Steckaufnahme 304, also an dem dem Schaft-Fußteil 22 zugewandten Ende des Kappenelements 30 ausgebildet. Entsprechend ist die Ausnehmung 245 im Abstand vom Schaft-Kopfende 25 angeordnet. Es wird eine Art Nut/Feder-Verbindung gebildet. Die Nut 245 ist so geformt, daß sie zum Fußende 22 des Schafts 2 hin mit Schrägfläche konisch verläuft. Dadurch entsteht am Schaft 2 eine konische Erweiterung, die genutzt werden kann, um in Einsteckrichtung, das heißt in Bewegungsrichtung des Kappenelements 30 auf das Schaft-Fußende 22 hin einen den Steckweg begrenzenden Anschlag zu bilden. Der Bördelrand 321 würde dann gegen die Schrägfläche zur Anlage kommen. Im Ausführungsbeispiel der Fig. 2 sind die Stecklänge des Steckabschnitts 24 und die durch die Nut 245 gebildete Taille jedoch so dimensioniert, daß die Stirnfläche des Schaft-Kopfteils 21 zur Stoßanlage gegen den den Anschlag 350 bildenden Bodenrand 35 kommt. Diese Anlage entsteht, wenn der Nagel 1 durch Eindrehen des Kappenelements 30 im Knochen verriegelt wird bzw. ist. Im verriegelten Zustand ist das Kappenelement 30 mit seinem Gewinde 33 in das Knochenmaterial vollständig hineingeschraubt. Die Nut 245 weist in Schaft-Axialrichtung eine Breite auf, die geringfügiges Axialspiel mit Anlage der Kopffläche des Schafts 2 gegen den Anschlag 350 zuläßt. Die Dimensionierung im Ausführungsbeispiel der Fig. 2 ist derart, daß in der Steckverbindung 31 axial nur geringes Spiel mit Spielpassung 37 zwischen dem Anschlag 350 und der Schaft-Stirnfläche vorgesehen ist. Man erkennt aus Fig. 2, daß der Nagelschaft 2 mittels der Sicherungs-Bördelverbindung 32 gegen axiale Bewegung in Richtung auf sein Fußende 22 gesichert ist. Bei einer solchen Bewegung kommt der Bördelrand 321 gegen einen kopfseitigen steilen Rand der Nut 245 zum Anschlag.

Im ganzen ist die Steckverbindung 31 des Nagels 1 gemäß Fig. 2 durch den langen Steckabschnitt 243 mit zugeordneter Steckaufnahme 304 und durch den kurzen Steckabschnitt 244 der Nut 245 mit zugeordneter, durch den Bördelrand 321 gebildeter Steckaufnahme 305 gebildet.

In dem Ausführungsbeispiel gemäß Fig. 3 umfaßt die Steckverbindung 31 eine Sicherungs-Verbindung 38. Letztere weist ein als Sprengring oder dergleichen Federring vorgesehenes elastisches Federelement 381 und an der Innenwand der Steckaufnahme 304 sowie korrespondierende, an dem Schaft-Steckabschnitt 24 umlaufend ausgebildete, Anschläge bildende halbkreisförmige Nuten 382 auf, die das Element 381 passend aufnehmen. Auf diese Weise ist die Steckverbindung 31 so gesichert, daß axiale Bewegung in und gegen Steckrichtung gesperrt wird. Insbesondere ist die Verbindung gegen Abziehen des Kappenelements 30 von dem Nagelschaft 2 gesichert. Das Federelement 381 sowie die Nuten 382 sind im Bereich des äußeren Drittels der Steckaufnahme 304, also zur Einsteckseite der Steckaufnahme 304 hin angeordnet. So ist die Sicherungs-Verbindung 38 an einer von dem Kopfende 25 des Nagelschafts 2 entfernt gelegenen, in axialer Richtung zum Schaft-Fußende 22 hin beabstandeten Umfangsstelle des Schaft-Steckabschnitts 243 zwischen diesem und dem Kappenelement 30 angeordnet und ausgebildet.

Sowohl der Bördelrand 321 in Fig. 2 als auch das Ring-Federelement 381 in Fig. 3 weisen jeweils eine Dimension auf, die in der Schaft-Axialrichtung kurz gegenüber der Stecklänge des Nagelschafts 2 in der Steckverbindung ist. Dadurch ist insbesondere erreicht, daß die Sicherungs-Verbindung 32 bzw. 38 die Drehlagerung des Kappenelements 30 auf dem Schaft 2 nicht beeinträchtigt. Dadurch, daß die Federelemente 321, 381 mit zugehörigen Nuten bzw. Ausnehmungen 245, 382 in Axialrichtung relativ kurz bemessen sind, weist der Nagelschaft 2 in dem überwiegenden größten Teil der Steckverbindung 31 den gleichen kreiszylindrischen Querschnitt wie in dem an die Steckverbindung 31 anschließenden, zum Schaft-Fußende 22 hin gelegenen langgestreckten Schaft-Abschnitt 26 auf. Dadurch ist insbesondere erreicht, daß der Nagelschaft 2 auch im Bereich der Steckverbindung 31 im wesentlichen die gleiche Biegesteifigkeit wie in dem an die Verbindung sich anschließenden Abschnitt aufweist.

Bei sämtlichen Ausführungsbeispielen wird stets gewährleistet, daß das Kappenelement 30 koaxial drehbar auf den Kopfteil 21 des Schafts 2 aufgesetzt ist. Von besonderem Vorteil ist, daß das Kappenelement 30 zum Verriegeln und/oder Plazieren des Nagels 1 weiter in Knochenmaterial geschraubt werden kann, ohne daß auf den Schaft 2 ein ihn mitdrehendes Moment ausgeübt wird.

## Patentansprüche

1. Intramedullärer Osteosynthese-Nagel (1) zum Versorgen von Röhrenknochenfrakturen, umfassend einen bohrungsfrei in Knochenmark einbringbaren, sich in Axialrichtung lang erstreckenden Schaft (2) mit einem Fußteil (22) und einem Kopfteil (21), ein zum Verriegeln des Nagels (1) im Knochen mit selbstschneidendem Außengewinde (33) versehenes Kappenelement (30) mit einer Aufnahme (304), mittels der es frei drehbar um die Längsachse (10) des Schaft-Kopfteils (21) auf diesen aufgesetzt ist, und eine Sicherungs-Verbindung (32, 38), die das Kappenelement (30) frei von Schraubverbindung unverlierbar mit dem Nagelschaft 2) derart verbindet, daß das Kappenelement (30) zum Setzen des Nagels (1) gegen axiales Abziehen von dem Kopfteil (21) des Nagelschafts (2) gesichert ist, wobei zwischen dem Kappenelement (30) und dem Kopfteil (21) des Schafts (2) eine die freie relative Drehung zwischen den beiden Teilen zulassende Steckverbindung (31) mit einem Steckabschnitt (243) des Schaft-Kopfteils (21) und zugeordneter Steckaufnahmen (304, 305) des Kappenelements (30) ausgebildet ist, **dadurch gekennzeichnet, daß** das Kappenelement (30) einen Kappen-Kopfteil (302) aufweist, der frei von der Sicherungs-Verbindung (32, 38) bleibt, wobei die Sicherungs-Verbindung (32, 38) an einer von dem Kopfende (25) des Nagelschafts (2) in axialer Richtung zum Schaft-Fußende (22) hin beabstandeten Umfangsstelle des Schaft-Steckabschnitts (243) zwischen diesem und dem Kappenelement (30) angeordnet und ausgebildet ist.

2. Nagel nach Anspruch 1, **dadurch gekennzeichnet, daß** das Kappenelement (30) einen außerhalb des Kopfteils (21) des Nagelschafts (2) sich erstreckenden schaftfreien Kappen-Kopfteil (302) zur Verbindung mit Drehwerkzeug sowie die den Schaft-Steckabschnitt (243) aufnehmende Steckaufnahme (304, 305) aufweist.

3. Nagel nach Anspruch 1 oder 2, **dadurch gekennzeichnet, daß** die von dem Schaft-Kopfende (25) beabstandete Sicherungs-Verbindung (32, 38) **dadurch** gebildet ist, daß sie wenigstens ein einen Verbindungs-Eingriff zwischen dem Kappenelement (30) und dem Nagelschaft (2) herstellendes, von dem Schaft-Kopfende (25) beabstandetes Federelement (321, 381) umfaßt, dessen Dimension in der Schaft-Axialrichtung kurz gegenüber der Stecklänge des Nagelschafts (2) in der Steckverbindung (31) ist.

4. Nagel nach einem der Ansprüche 1 bis 3, **dadurch gekennzeichnet, daß** die Sicherungs-Verbindung (32, 38) wenigstens einen an dem Kappenelement (30) angeordneten Vorsprung (321, 381) und eine an dem Nagelschaft (2) ausgebildete zugeordnete Ausnehmung (245, 382) aufweist.

5. Nagel nach Anspruch 3 oder 4, **dadurch gekennzeichnet, daß** die Sicherungs-Verbindung (32) ein Federelement in Form eines Bördelrandes (321) aufweist, der an dem dem Schaft-Fußteil (22) zugewandten Ende des Kappenelements (30) ausgebildet ist und in eine zugeordnete, vom Schaft-Kopfende (25) entfernt gelegene sowie am Nagelschaft (2) ausgebildete Ausnehmung (245) eingreift.

6. Nagel nach Anspruch 3 oder 4, **dadurch gekennzeichnet, daß** die Sicherungs-Verbindung (38) durch wenigstens ein sowohl in den Nagelschaft (2) als auch in das Kappenelement (30) eingreifendes Federelement (381) gebildet ist.

7. Nagel nach Anspruch 6, **dadurch gekennzeichnet, daß** die Sicherungs-Verbindung (38) im Bereich des äußeren Drittels der Steckaufnahme (304) der Steckverbindung (31) ausgebildet ist.

8. Nagel nach einem der Ansprüche 1 bis 7, **dadurch gekennzeichnet, daß** die Sicherungs-Verbindung (32) mit Spielpassung (37) derart ausgebildet ist, daß in der unverlierbaren Steckverbindung (31) in Axialrichtung des Nagelschafts (2) relatives Bewegungsspiel zwischen dem Kappenelement (30) und dem Nagelschaft (2) bleibt.

9. Nagel nach Anspruch 8, **dadurch gekennzeichnet, daß** die Sicherungs-Verbindung (32) durch Federelement (321) und zugeordnete, am Nagelschaft (2) ausgebildete Nut (245) gebildet ist, die in Schaft-Axialrichtung eine das Axialspiel zulassende Breite aufweist.

10. Nagel nach Anspruch 9, **dadurch gekennzeichnet, daß** sich der Nagelschaft (2) längs der Nut (245) konisch erweitert, so daß ein die Spiel-Bewegung des Kappenelements (30) in Richtung zum Schaft-Fußende (22) hin begrenzender Anschlag ausgebildet ist.

11. Nagel nach einem der Ansprüche 1 bis 10, **dadurch gekennzeichnet, daß** das Kappen-Kopfteil (302) als Hohlkopf mit Durchgangsloch (36) zwischen dem Kappen-Kopfteil (302) und der Steckaufnahme (304) ausgebildet ist.

12. Nagel nach Anspruch 11, **dadurch gekennzeichnet, daß** das Durchgangsloch (36) einen der Steckaufnahme (304) zugewandten umlaufenden Rand aufweist, der in der Steckverbindung (31) einen Bodenanschlag (35) für die Kopf-Endfläche des Nagelschafts (2) bildet.

13. Nagel nach Anspruch 11 oder 12, **dadurch gekennzeichnet, daß** der Kappen-Hohlkopf mit mehrkantiger Aussparung (39) zur Verbindung mit Drehwerkzeug ausgebildet ist.

14. Nagel nach einem der Ansprüche 11 bis 13, **dadurch gekennzeichnet, daß** der Kappen-Hohlkopf eine umlaufende Kappenwand (361) aufweist, die stärker als die umlaufende Wand (306) der angrenzenden Steckaufnahme (304) ausgebildet ist.

15. Nagel nach einem der Ansprüche 1 bis 14, **dadurch gekennzeichnet, daß** das Kappenelement (30) einen den Kappen-Kopfteil (21) von der Steckaufnahme (304, 305) abgrenzenden Boden (35) aufweist und eine Nut/Feder-Verbindung (321, 245) der Sicherungs-Verbindung (32) so ausgebildet ist, daß die Endfläche des Schaftkopfes (21) an dem Boden (35) zur Stoßanlage kommt.

16. Nagel nach einem der Ansprüche 1 bis 15, **dadurch gekennzeichnet, daß** der Schaft-Kopfteil (21) wenigstens zwei Steckabschnitte (243, 244) und das Kappenelement (30) für jeden Steckabschnitt (243, 244) eine zugeordnete Steckaufnahme (304, 305) aufweist.

17. Nagel nach einem der Ansprüche 1 bis 16, **dadurch gekennzeichnet, daß** das selbstschneidende Außengewinde (33) des Kappenelements (30) längs dessen gesamter Axiallänge ausgebildet ist.

18. Nagel nach einem der Ansprüche 1 bis 17, **dadurch gekennzeichnet, daß** der Nagelschaft (2) wenigstens in dem größten Teil der Steckverbindung (31) den gleichen Querschnitt wie in einem an die Steckverbindung (31) anschließenden, zum Schaft-Fußende (22) hin gelegenen langgestreckten Schaft-Abschnitt (26) aufweist.

## Claims

1. Intramedullary osteosynthesis pin (1) for treatment of long bone fractures, comprising a shaft (2) which can be introduced without a bore into bone marrow and extending along in axial direction and having a foot part (22) and a head part (21), a cap element (30) provided with self-cutting external thread (33) for locking the pin (1) in the bone and having a recess (304), by means of which it is placed to be freely rotatable about the longitudinal axis (10) of the shaft head part (21) on the latter, and a locking connection (32, 38) which connects the cap element (30) free of screw connection undetachably to the pin shaft (2), such that the cap element (30) for placing the pin (1) is secured against axial removal from the head part (21) of the pin shaft (2), wherein between the cap element (30) and the head part (21) of the shaft (2), a plug connection (31) permitting the free relative rotation between the two parts and with a plug section (243) of the shaft head part (21) and assigned plug recesses (304, 305) of the cap element (30) is formed, **characterized in that** the cap element (30) has a cap head part (302), which remains free of the locking connection (32, 38), wherein the locking connection (32, 38) is arranged and formed on a peripheral point of the shaft plug section (243) spaced from the head end (25) of the pin shaft (2) in axial direction towards the shaft foot end (22) between the shaft plug section (243) and the cap element (30).

2. Pin according to claim 1, **characterized in that** the cap element (30) has a shaft-free cap head part (302) extending outside of the head part (21) of the pin shaft (2) for connection by a screwdriver tool and the plug recess (304, 305) receiving the shaft plug section (243).

3. Pin according to claim 1 or 2, **characterized in that** the locking connection (32, 38) spaced from the shaft head end (25) is formed **in that** it comprises at least one key element (321, 381) producing a connection engagement between the cap element (30) and the pin shaft (2) and spaced from the shaft head end (25), the dimension of which key element (321, 381) in the shaft axial direction being short with respect to the plug length of the pin shaft (2) in the plug connection (31).

4. Pin according to any one of claims 1 to 3, **characterized in that** the locking connection (32, 38) has at least one projection (321, 381) arranged on the cap element (30) and an assigned recess (245, 382) formed on the pin shaft (2).

5. Pin according to claim 3 or 4, **characterized in that** the locking connection (32) has a key element in the form of a beaded edge (321), which is formed on the end of the cap element (30) facing the shaft foot part (22) and engages in an assigned recess (245) placed remote from the shaft head end (25) and formed on the pin shaft (2).

6. Pin according to claim 3 or 4, **characterized in that** the locking connection (38) is formed by at least one key element (381) engaging both in the pin shaft (2) and in the cap element (30).

7. Pin according to claim 6, **characterized in that** the locking connection (38) is formed in the region of the outer third of the plug recess (304) of the plug connection (31).

8. Pin according to any one of claims 1 to 7, **characterized in that** the locking connection (32) is formed with clearance fit (37) such that in the undetachable plug connection (31) in axial direction of the pin shaft (2), relative movement clearance remains between the cap element (30) and the pin shaft (2).

9. Pin according to claim 8, **characterized in that** the locking connection (32) is formed by key element (321) and assigned groove (245) formed on the pin shaft (2) and which has a width permitting the axial clearance in axial direction of the shaft.

10. Pin according to claim 9, **characterized in that** the pin shaft (2) expands conically along the groove (245), so that a stop limiting the clearance movement of the cap element (30) in the direction towards the shaft foot end (22) is formed.

11. Pin according to any one of claims 1 to 10, **characterized in that** the cap head part (302) is formed as a hollow head with passage hole (36) between the cap head part (302) and the plug recess (304).

12. Pin according to claim 11, **characterized in that** the passage hole (36) has a circumferential edge facing the plug recess (304) and which forms a base stop (35) for the head end surface of the pin shaft (2) in the plug connection (31).

13. Pin according to claim 11 or 12, **characterized in that** the cap hollow head having a many-sided recess (39) is formed for connection with a screwdriver tool.

14. Pin according to any one of claims 11 to 13, **characterized in that** the cap hollow head has a circumferential cap wall (361) which is designed to be thicker than the circumferential wall (306) of the adjoining plug recess (304).

15. Pin according to any one of claims 1 to 14, **characterized in that** the cap element (30) has a base (35) delimiting the cap head part (21) from the plug recess (304, 305) and a key/groove connection (321, 245) of the locking connection (32) is designed so that the end surface of the shaft head (21) comes to an abutting rest against the base (35).

16. Pin according to any one of claims 1 to 15, **characterized in that** the shaft head part (21) has at least two plug sections (243, 244) and the cap element (30) for each plug section (243, 244) has an assigned plug recess (304, 305).

17. Pin according to any one of claims 1 to 16, **characterized in that** the self-cutting external thread (33) of the cap element (30) is formed along its entire axial length.

18. Pin according to any one of claims 1 to 17, **characterized in that** the pin shaft (2) at least in the largest part of the plug connection (31) has the same cross-section as in an elongated shaft section (26) following the plug connection (31) and lying towards the shaft foot end (22).

## Revendications

1. Clou (1) d'ostéosynthèse intramédullaire pour soigner des fractures d'os longs, comprenant une hampe (2) insérable sans perçage dans la moelle osseuse, s'étendant en long dans le sens axial, avec une base (22) et un sommet (21), un embout (30) pourvu d'un filet extérieur (33) autotaraudeur pour fixer le clou (1) dans l'os, avec un logement (304) au moyen duquel il est posé sur celui-ci de façon à pouvoir tourner librement autour de l'axe longitudinal (10) du sommet (21) de la hampe, et un raccordement (32, 38) de sécurité qui relie l'embout (30) à la hampe (2) du clou de façon imperdable sans raccord fileté, de sorte que l'embout (30) pour poser le clou (1) soit assuré contre un retrait axial du sommet (21) de la hampe (2) du clou, un raccord (31) à emboîtement étant constitué entre l'embout (30) et le sommet (21), autorisant une rotation relative libre entre les deux parties, avec une section (243) à emboîtement du sommet (21) de la hampe et des logements (304, 305) dédiés de l'embout (30), **caractérisé en ce que** l'embout (30) présente une tête (302) qui reste libérée du raccordement (32, 38) de sécurité, le raccordement (32, 38) de sécurité étant disposé et constitué à un endroit périphérique de la section (243) à emboîtement de la hampe, espacé de l'extrémité haute (25) de la hampe (2) du clou dans le sens axial vers l'extrémité basse (22) de la hampe, entre celle-ci et l'embout (30).

2. Clou selon la revendication 1, **caractérisé en ce que** l'embout (30) présente une tête (302) libérée de la hampe, s'étendant à l'extérieur du sommet (21) de la hampe (2) du clou pour raccorder un outil rotatif, ainsi que le logement (304, 305) recevant la section (243) à emboîtement de la hampe.

3. Clou selon la revendication 1 ou 2, **caractérisé en ce que** le raccordement (32, 38) de sécurité, espacé de l'extrémité haute (25) de la hampe, est constitué de telle sorte qu'il comprend au moins un élément (321, 381) à ressort produisant un engagement de raccordement entre l'embout (30) et la hampe (2) du clou, dont la dimension dans le sens axial de la hampe est courte par rapport à la longueur d'emboîtement de la hampe (2) du clou dans le raccord (31) à emboîtement.

4. Clou selon l'une des revendications 1 à 3, **caractérisé en ce que** le raccordement (32, 38) de sécurité présente au moins un ressaut (321, 381) aménagé sur l'embout (30) et un creux (245, 382) dédié, formé sur la hampe (2) du clou.

5. Clou selon la revendication 3 ou 4, **caractérisé en ce que** le raccordement (32) de sécurité présente un élément à ressort sous la forme d'un bord relevé (321) qui est formé à l'extrémité de l'embout (30) tournée vers la base (22) de la hampe et s'engage dans un creux (245) dédié, situé à distance de l'extrémité haute (25) de la hampe et aménagé sur la hampe (2) du clou.

6. Clou selon la revendication 3 ou 4, **caractérisé en ce que** le raccordement (38) de sécurité est constitué par au moins un élément (381) à ressort s'engageant à la fois dans la hampe (2) du clou et dans l'embout (30).

7. Clou selon la revendication 6, **caractérisé en ce que** le raccordement (38) de sécurité est formé dans la zone du tiers extérieur du logement (304) du raccord (31) à emboîtement.

8. Clou selon l'une des revendications 1 à 7, **caractérisé en ce que** le raccordement (32) de sécurité est ajusté avec du jeu (37), de sorte qu'il reste un jeu relatif dans le raccord (31) à emboîtement imperdable dans le sens axial de la hampe (2) du clou, entre l'embout (30) et la hampe (2) du clou.

9. Clou selon la revendication 8, **caractérisé en ce que** le raccordement (32) de sécurité est constitué par un élément (321) à ressort et une rainure (245) formée sur la hampe (2) du clou, qui présente, dans le sens axial de la hampe, une largeur autorisant le jeu axial.

10. Clou selon la revendication 9, **caractérisé en ce que** la hampe (2) du clou s'élargit de manière conique le long de la rainure (245), de sorte qu'une butée limitant le jeu de l'embout (30) soit constituée dans le sens vers la base (22) de la hampe.

11. Clou selon l'une des revendications 1 à 10, **caractérisé en ce que** la tête (302) de l'embout est formée en tant que tête creuse avec un trou débouchant (36) entre la tête (302) de l'embout et le logement (304).

12. Clou selon la revendication 11, **caractérisé en ce que** le trou débouchant (36) présente un bord circulaire tourné vers le logement (304), qui forme, dans le raccord (31) à emboîtement, une butée (35) de fond pour la face de l'extrémité haute de la hampe (2) du clou.

13. Clou selon la revendication 11 ou 12, **caractérisé en ce que** la tête creuse de l'embout est constitué avec une cavité (39) à bords multiples pour raccorder l'outil rotatif.

14. Clou selon l'une des revendications 11 à 13, **caractérisé en ce que** la tête creuse de l'embout présente une paroi périphérique (361) qui est plus épaisse que la paroi périphérique (306) du logement (304) adjacent.

15. Clou selon l'une des revendications 1 à 14, **caractérisé en ce que** l'embout (30) présente un fond (35) démarquant le sommet (21) de l'embout du logement (304, 305) et **en ce qu'**un raccord rainure/ressort (321, 245) du raccordement (32) de sécurité est constitué, de sorte que la face de l'extrémité du sommet (21) de la hampe arrive en position de butée sur le fond (35).

16. Clou selon l'une des revendications 1 à 15, **caractérisé en ce que** le sommet (21) de la hampe présente au moins deux sections (243, 244) à emboîtement et l'embout (30) un logement (304, 305) dédié à chaque section (243, 244) à emboîtement.

17. Clou selon l'une des revendications 1 à 16, **caractérisé en ce que** le filet extérieur (33) autotaraudeur de l'embout (30) est formé tout le long de sa longueur axiale.

18. Clou selon l'une des revendications 1 à 17, **caractérisé en ce que** la hampe (2) du clou présente, au moins dans la majeure partie du raccord (31) à emboîtement, la même coupe transversale que dans une section (26) étendue de la hampe, subséquente au raccord (31) à emboîtement et allant vers l'extrémité basse (22) de la hampe.
